# EUROPÄISCHE PATENTSCHRIFT

(11) **EP 2 262 544 B1**
(45) Veröffentlichungstag und Bekanntmachung des Hinweises auf die Patenterteilung: **17.08.2011**
(21) Anmeldenummer: 09726079.8
(22) Anmeldetag: 10.03.2009
(51) Int. Cl.: A61L 2/20, B65B 55/10

(54) **GEFÄSSBEHANDLUNGSMASCHINE**
VESSEL TREATMENT MACHINE
MACHINE DE TRAITEMENT DE RÉCIPIENTS

(30) Priorität: 25.03.2008 DE 102008015675
(43) Veröffentlichungstag der Anmeldung: 22.12.2010
(73) Patentinhaber: KHS GmbH, 44143 Dortmund (DE)
(72) Erfinder: BAUER, Eva, 21337 Lüneburg (DE); BEHRENDT, Frank, Peter, 25421 Pinneberg (DE); KIEFER, Margit, 20095 Hamburg (DE); KLEISS, Andreas, 22846 Norderstedt (DE); NEUMANN, Bernd, 22159 Hamburg (DE); STANISLAWSKI, Andreas, 22119 Hamburg (DE); STUWE, Hans, Peter, 45699 Herten (DE)
(86) Internationale Anmeldenummer: PCT/EP2009/001697
(87) Internationale Veröffentlichungsnummer: WO 2009/118096

(56) Entgegenhaltungen:
- EP-A- 0 353 486
- EP-A- 0 427 051
- WO-A-02/074351
- WO-A-03/030950
- DE-B4-102004 029 803

## Beschreibung

Die Erfindung betrifft eine Gefäßbehandlungsmaschine zur Sterilisation von Behältern mittels einer Sterilisationsflüssigkeit, mit wenigstens einer Flüssigkeits-Zuleitung und einer oder mehreren Gas-Zuleitungen, ferner mit zumindest einer Mischkammer zur Herstellung eines Flüssigkeits-/Gasgemisches, und mit wenigstens einem Ventil in der Flüssigkeits-Zuleitung.

Eine solche Gefäßbehandlungsmaschine wird im Großen und Ganzen in der WO 03/030950 A1 beschrieben. Ähnliche Ausgestaltungen sind Gegenstand der DE 10 2004 029 803 B4. Bei der Gefäßbehandlungsmaschine handelt es sich regelmäßig um eine solche, die in der Getränkeindustrie eingesetzt wird, d. h. beim Abfüllen von Getränken in Flaschen, Dosen oder allgemein Behältern aus beispielsweise Kunststoff, Metall, Glas usw. zum Einsatz kommt.

Meistens sind solche Gefäßbehandlungsmaschinen einer Abfüllanlage vorgeschaltet und dienen dazu, die erforderliche Keimfreiheit innerhalb des Behälters und damit die Haltbarkeit des abgefüllten Produktes sicherzustellen. Zu diesem Zweck wird regelmäßig flüssiges Wasserstoffperoxid (H₂O₂) als Sterilisationsflüssigkeit fein verstäubt einem Luftstrom in der Mischkammer beigemischt. Im Anschluss daran mag dieses Flüssigkeits-/Gasgemisch bzw. Wasserstoffperoxid-/Luftgemisch einem Verdampfer zugeführt werden, in welchem das noch flüssige H₂O₂ vollständig verdampft wird.

Tatsächlich ist es nämlich für die Aktivierung des Wasserstoffperoxides, d. h. für seine Zersetzung, erforderlich, dieses auf eine bestimmte Temperatur zu erwärmen. Bei dieser Temperatur zerfällt das Wasserstoffperoxid in Wasser und freie Radikale, welche für die eigentliche Sterilisation verantwortlich zeichnet. Das hat sich bewährt, und zwar auch bei der Sterilisation von Pappbehältern, wie dies grundsätzlich in der US 6 120 730 beschrieben wird.

Die einwandfreie Sterilisation des Behälters setzt voraus, dass die Sterilisationsflüssigkeit wohl dosiert zur Verfügung gestellt wird. Das geschieht meistens mit Hilfe des in der Flüssigkeits-Zuleitung vorhandenen wenigstens einen Ventils. Allerdings ergibt sich hier das Problem, dass die vom Ventil an beispielsweise eine Zerstäubungseinrichtung weitergeleitete Flüssigkeitsmenge von Fall zu Fall variieren kann. Diese variierende Flüssigkeitsmenge erklärt sich aufgrund von in dem Ventil verbleibender Sterilisationsflüssigkeit von einem zum anderen Abgabezyklus an einen Behandlungskopf. Darüber hinaus müssen eventuelle weitere sogenannte "Totmengen" in einer Zuleitung vom Ventil zur Mischkammer bzw. bis hin zum Zerstäuber berücksichtigt werden. - Hier setzt die Erfindung ein.

Der Erfindung liegt das technische Problem zugrunde, eine Gefäßbehandlungsmaschine zur Sterilisation von Behältern mittels einer Sterilisationsflüssigkeit so weiter zu entwickeln, dass die Sterilisationsflüssigkeit in exakt dosierbaren und reproduzierbaren Mengen innerhalb der Mischkammer zur Verfügung steht.

Zur Lösung dieser technischen Problemstellung ist bei einer gattungsgemäßen Gefäßbehandlungsmaschine vorgesehen, dass die Mischkammer und eine ausgangsseitige Ventilkammer des Ventils funktional zusammenfallen. D. h., die am Ausgang des Ventils in der Flüssigkeits-Zuleitung obligatorische Ventilkammer wird zugleich als Mischkammer genutzt, d. h. für die Herstellung des Flüssigkeits-/Gasgemisches.

Auf diese Weise sorgt das in die Mischkammer bzw. die Ventilkammer eintretende Gas automatisch dafür, dass die Ventilkammer gleichsam gespült wird, und zwar in dem gewünschten Mischungsverhältnis. Dazu mündet die Gas-Zuleitung für die Zufuhr des Gases üblicherweise unmittelbar in die ausgangsseitige Ventilkammer. D. h., die Ventilkammer wird zur Verdüsung des Flüssigkeits-/Gasgemisches genutzt und dadurch nach jedem Hub entleert.

Ein solcher Hub korrespondiert zur erforderlichen Menge an Flüssigkeit und Gas, die für die Behandlung meistens eines Behälters erforderlich ist. Da sowohl die Sterilisationsflüssigkeit als auch das Gas unter Druck zugeführt werden und auch die Gas-Zuleitung mit einem Ventil ausgerüstet ist, kann durch entsprechende Ansteuerung der Ventile für die jeweilige Herstellung des Flüssigkeits-/Gasgemisches innerhalb der Mischkammer respektive Ventilkammer gesorgt werden. Dieses Flüssigkeits-/Gasgemisch wird danach dem Behandlungskopf zugeführt, in dem in der Regel der bereits beschriebene Verdampfungsvorgang und damit die Aktivierung der Sterilisationsflüssigkeit erfolgt. Die aktivierte Sterilisationsflüssigkeit bzw. das Gemisch wird abschließend in den Behälter eingeblasen. Damit ist ein Hub bzw. Arbeitshub beendet.

Da erfindungsgemäß der Dosiervorgang vollständig innerhalb der die Mischkammer darstellenden ausgangsseitigen Ventilkammer des Ventils in der Flüssigkeits-Zuleitung stattfindet, ist die Dosierung bzw. der Dosiervorgang vom Verdampfungsvorgang getrennt und somit nicht zeitkritisch. Dadurch kann die Dosiermenge für die Sterilisationsflüssigkeit besonders feinfühlig vorgegeben werden, beispielsweise im Sinne einer Regelung. Immer ist gewährleistet, dass das anschließend die ausgangsseitige Ventilkammer bzw. Mischkammer verlassende Flüssigkeits-/Gasgemisch zielgenau an den zu behandelnden Behälter angepasst ist.

Das mag im Detail dergestalt erfolgen, dass ein Ventilkörper innerhalb des Ventils in der Flüssigkeits-Zuleitung eine Öffnung zwischen einer eingangsseitigen Ventilkammer und der ausgangsseitigen Ventilkammer mehr oder minder öffnet. Wenn bei diesem Vorgang die Stellung des Ventilkörpers abgefragt wird, kann die in die ausgangsseitige Ventilkammer dosierte Menge an Sterilisationsflüssigkeit exakt im Sinne einer Regelung vorgegeben werden. Das geschieht solange, bis die auf diese Weise in der ausgangsseitigen Ventilkammer vorhandene Menge der Sterilisationsflüssigkeit für die Behandlung des zugehörigen Behälters und die anschließende Verdüsung mit Hilfe des Gases ausreichend ist.

Dann mag der Ventilkörper zwischen den beiden Ventilkammern geschlossen werden und kann anschließend das Ventil in der Gaszuleitung geöffnet werden, um die innerhalb der ausgangsseitigen Ventilkammer vorhandene Menge an Sterilisationsflüssigkeit zu verdüsen. Weil dieser Vorgang unmittelbar in der ausgangsseitigen Ventilkammer stattfindet, die zugleich als Mischkammer fungiert, ist sichergestellt, dass bei dem beschriebenen Verdüsungsvorgang sämtliche Flüssigkeitsbestandteile der Sterilisationsflüssigkeit Eingang in das Flüssigkeits-/Gasgemisch finden. Etwaige Totmengen wie beim Stand der Technik werden nicht beobachtet.

Weitere vorteilhafte Ausgestaltungen werden im Folgenden beschrieben. So ist es denkbar, die ausgangsseitige Ventilkammer des in der Flüssigkeits-Zuleitung befindlichen Ventils respektive Regelventils unmittelbar an einen Behandlungskopf anzuschließen. Es ist aber auch möglich, dass das in der ausgangsseitigen Ventilkammer erzeugte Flüssigkeits-/Gasgemisch einer weiteren zweiten Mischkammer zugeführt wird, die dann ihrerseits an den Behandlungskopf angeschlossen ist. D. h., der Mischvorgang findet zweistufig statt, indem zunächst das Flüssigkeits-/Gasgemisch in der ausgangsseitigen Ventilkammer bzw. ersten Mischkammer hergestellt wird und dann dieses Flüssigkeits-/Gasgemisch in eine weitere zweite Mischkammer überführt wird, die (ebenfalls) mit einer weiteren zweiten Gaszuleitung beaufschlagt wird. Dadurch kann der Verdüsungsvorgang optimiert werden. D.h., in diesem Fall wird die weitere zweite Mischkammer von der weiteren zweiten Gaszuleitung und einer Kammerzuleitung gespeist, welche die erste Mischkammer bzw. ausgangsseitige Ventilkammer mit der zweiten Mischkammer verbindet.

Um den Mischvorgang noch weiter zu beschleunigen, ist es denkbar, dass die Gas-Zuleitung mit einer Venturidüse oder allgemein einer Düse eingangsseitig der (jeweiligen) Mischkammer ausgerüstet ist. - Wie bereits erläutert, handelt es sich auch bei dem Ventil in der Gas-Zuleitung um ein Regelventil, also ein solches Ventil, bei dem die Stellung eines Ventilkörpers erfasst und im Sinne einer Regelung so eingestellt werden kann, dass exakt die gewünschte Menge an dosiertem Gas bzw. dosierter Flüssigkeit (beim Regelventil in der Flüssigkeits-Zuleitung) das Ventil verlässt.

Im Allgemeinen handelt es sich bei der Sterilisationsflüssigkeit um flüssiges Wasserstoffperoxid, was jedoch nicht als zwingend anzusehen ist. Als Gas kommt überwiegend Sterilluft zum Einsatz, also Luft, die zuvor sterilisiert worden ist. **-** Gegenstand der Erfindung ist auch ein Verfahren zur Sterilisation von Behältern nach Anspruch 10. Das beschriebene Verfahren kann dabei in der im Detail erläuterten Gefäßbehandlungsmaschine durchgeführt werden. Das ist jedoch nicht zwingend.

Im Folgenden wird die Erfindung anhand einer lediglich ein Ausführungsbeispiel darstellenden Zeichnung näher erläutert; es zeigen:
- **Fig. 1**: eine erfindungsgemäße Gefäßbehandlungsmaschine, reduziert auf die für die Erfindung wesentlichen Bestandteile in einer ersten Ausgestaltung und
- **Fig. 2**: den Gegenstand nach Fig. 1 in einer Variante.

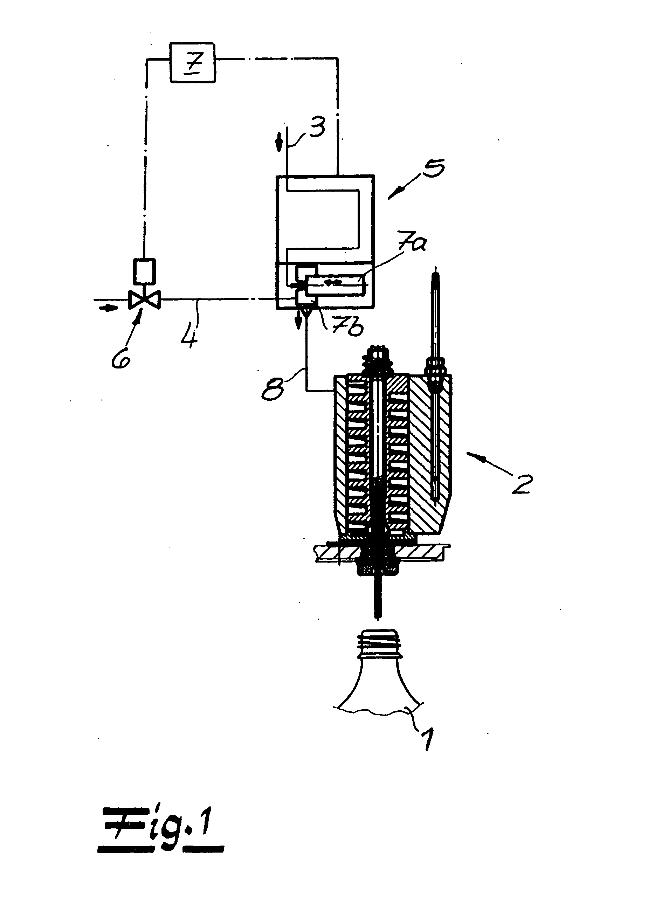

In den Figuren ist eine Gefäßbehandlungsmaschine dargestellt, die zur Sterilisation von Behältern 1 dient. Bei dem lediglich in Fig. 1 angedeuteten Behälter 1 mag es sich um eine Kunststoff-PET-Flasche handeln, was selbstverständlich nicht einschränkend zu verstehen ist. Die Sterilisation erfolgt mit Hilfe eines Flüssigkeits-/Gasgemisches, welches in einem Behälterkopf 2 aktiviert werden mag, wie dies im Detail in der DE 10 2004 029 803 B4 beschrieben wird. Dazu ist der Behälterkopf 2 mit beispielsweise einem Wärmetauscher ausgerüstet, welcher dafür sorgt, dass die Sterilisationsflüssigkeit im Beispielfall aktiviert wird.

Tatsächlich handelt es sich bei der Sterilisationsflüssigkeit vorliegend und nicht einschränkend um flüssiges Wasserstoffperoxid, welches insgesamt über eine Flüssigkeits-Zuleitung 3 zugeführt wird. Diese Sterilisationsflüssigkeit bzw. das flüssige Wasserstoffperoxid wird mit einem Gas (Sterilluft) gemischt, welches mit Hilfe einer Gas-Zuleitung 4 zugeführt wird. Innerhalb der Flüssigkeits-Zuleitung 3 ist ein Ventil 5 vorgesehen, welches als Regelventil ausgestaltet ist. Auch die Gas-Zuleitung 4 verfügt über ein Ventil 6, das ebenfalls als Regelventil 6 ausgelegt ist. Beide Regelventile 5, 6 sind an eine gemeinsame Steuereinheit 7 angeschlossen, welche die Stellung ihrer jeweiligen Ventilkörper abfragt, um im Sinne einer Regelung eine dosierte Menge an Sterilisationsflüssigkeit ausgangsseitig des Ventils 5 und im Übrigen eine dosierte Menge an Gas ausgangsseitig des Ventils 6 zur Verfügung zu stellen. Bei dem Gas handelt es sich nicht einschränkend um Sterilluft.

Man erkennt, dass das Ventil bzw. Regelventil 5 mit einer eingangsseitigen Ventilkammer 7a und einer ausgangsseitigen Ventilkammer 7b ausgerüstet ist, die voneinander durch einen Ventilkörper getrennt sind. Je nach Stellung dieses Ventilkörpers wird die Menge an Sterilisationsflüssigkeit innerhalb der ausgangsseitigen Ventilkammer 7b vorgegeben, und zwar - wie beschrieben - im Sinne einer Regelung, die mit Hilfe der Steuereinheit 7 überprüft und vorgegeben wird. Sobald in der ausgangsseitigen Ventilkammer 7b die erforderliche dosierte Menge an Sterilisationsflüssigkeit vorhanden ist, wird im Allgemeinen der Ventilkörper geschlossen und im Anschluss hieran das Ventil 6 geöffnet.

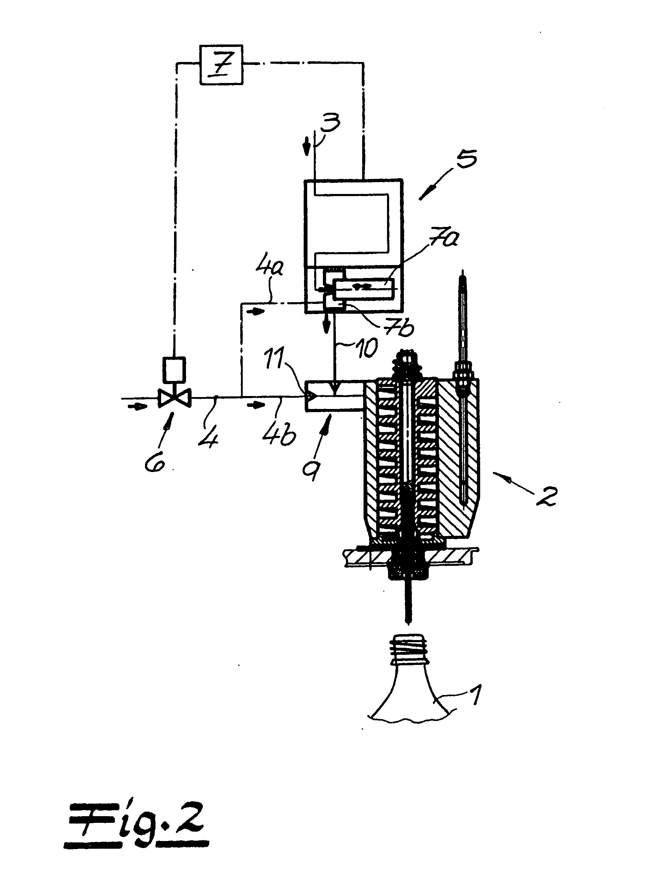

Da die Gas-Zuleitung 4 an die ausgangsseitige Ventilkammer 7b angeschlossen ist bzw. in diese mündet, übernimmt die ausgangsseitige Ventilkammer 7b zugleich die Funktion einer Mischkammer 7b zur Herstellung des Flüssigkeits-/Gasgemisches bzw. bei der Verdüsung der Sterilisationsflüssigkeit im Innern der ausgangsseitigen Ventilkammer 7b mit Hilfe des zugeführten Gases. D. h., die Mischkammer 7b und die ausgangsseitige Ventilkammer 7b fallen funktional zusammen. Man erkennt, dass im Rahmen der Variante nach der Fig. 1 die ausgangsseitige Ventilkammer bzw. Mischkammer 7b unmittelbar an den Behandlungskopf 2 angeschlossen ist, nämlich über eine Ausgangsleitung 8. Dagegen ist bei der Variante nach Fig. 2 eine weitere Mischkammer 9 realisiert.

Tatsächlich teilt sich die Gas-Zuleitung 4 nach dem Ventil 6 in zwei separate Gas-Zuleitungen 4a, 4b, die in die jeweiligen Mischkammern 7b respektive 9 bei der Variante nach der Fig. 2 münden. Dabei übernimmt die Mischkammer 7b bzw. ausgangsseitige Ventilkammer 7b des Ventils 5 die Funktion einer ersten Mischkammer 7b, wohingegen die weitere Mischkammer 9 als zweite Mischkammer fungiert. Das innerhalb der ersten Mischkammer 7b erzeugte Flüssigkeits-/Gasgemisch wird über eine Kammerzuleitung 10 in die zweite Mischkammer 9 gespeist und erfährt hier eine weitere Mischung dergestalt, dass die weitere Gas-Zuleitung 4b an die zweite Mischkammer 9 angeschlossen ist.

Dabei sorgt eine Venturidüse 11 eingangsseitig der zweiten Mischkammer 9 dafür, dass das über die Gas-Zuleitung 4b in die Mischkammer 9 zugeführte Gas eine Beschleunigung erfährt, so dass die Verdüsung in der zweiten Mischkammer 9 intensiviert wird. Die zweite Mischkammer 9 ist unmittelbar an den Behandlungskopf 2 angeschlossen, was selbstverständlich nicht als zwingend anzusehen ist. Der Behandlungskopf 2 mag selbst Bestandteil einer Rundläufer- oder Linearmaschine sein, wie dies allgemein bekannt ist.

Man erkennt schlussendlich, dass die Sterilisationsflüssigkeit innerhalb der Flüssigkeits-Zuieitung 3 zusätzlich das natürliche Gefälle ausnutzt, weil die Flussrichtung der Sterilisationsflüssigkeit der Schwerkraft folgt. Dadurch lässt sich eine optimale Entgasung der Sterilisationsflüssigkeit erreichen, bevor diese mit dem Gas in der Mischkammer bzw. ersten Mischkammer 7b gemischt wird. Dadurch ist gewährleistet, dass sich das Flüssigkeits-/Gasgemisch praktisch nur aus den beiden Bestandteilen der Sterilisationsflüssigkeit und dem Gas (Sterilluft) zusammensetzt.

## Patentansprüche

1. Gefäßbehandlungsmaschine zur Sterilisation von Behältern (1) mittels einer Sterilisationsflüssigkeit, mit wenigstens einer Flüssigkeits-Zuleitung (3) und einer oder mehreren Gas-Zuleitungen (4; 4a, 4b), ferner mit zumindest einer Mischkammer (7b) zur Herstellung eines Flüssigkeits-/Gasgemisches, und mit wenigstens einem Ventil (5) in der Flüssigkeits-Zuleitung (3), **dadurch ge-kennzeichnet**, dass die Mischkammer (7b) und eine ausgangsseitige Ventilkammer (7b) des Ventils (5) funktional zusammenfallen.

2. Gefäßbehandlungsmaschine nach Anspruch 1, **dadurch gekennzeichnet, dass** die Gas-Zuleitung (4; 4a, 4b) in die ausgangsseitige Ventilkammer (7b) mündet.

3. Gefäßbehandlungsmaschine nach Anspruch 1 oder 2, **dadurch gekennzeichnet, dass** die ausgangsseitige Ventilkammer (7b) unmittelbar an einen Behandlungskopf (2) angeschlossen ist.

4. Gefäßbehandlungsmaschine nach einem der Ansprüche 1 bis 3, **dadurch gekennzeichnet, dass** die ausgangsseitige Ventilkammer (7b) unter Zwischenschaltung einer weiteren zweiten Mischkammer (9) an den Behandlungskopf (2) angeschlossen ist.

5. Gefäßbehandlungsmaschine nach Anspruch 4, **dadurch gekennzeichnet, dass** die weitere zweite Mischkammer (9) von einer weiteren zweiten Gaszuleitung (4b) und einer Kammerzuleitung (10) gespeist wird.

6. Gefäßbehandlungsmaschine nach einem der Ansprüche 1 bis 5, **dadurch gekennzeichnet, dass** die Gas-Zuleitung (4; 4a, 4b) eine Venturidüse (11) zur Beschleunigung der Fließgeschwindigkeit des Gases aufweist.

7. Gefäßbehandlungsmaschine nach einem der Ansprüche 1 bis 6, **dadurch gekennzeichnet, dass** das Ventil (5) in der Flüssigkeits-Zuleitung (3) als Regelventil ausgeführt ist.

8. Gefäßbehandlungsmaschine nach einem der Ansprüche 1 bis 7, **dadurch gekennzeichnet, dass** die Gas-Zuleitung (4) ein Ventil (6), insbesondere Regelventil (6), aufweist.

9. Gefäßbehandlungsmaschine nach einem der Ansprüche 1 bis 8, **dadurch gekennzeichnet, dass** die Sterilisationsflüssigkeit als flüssiges H₂O₂ und das Gas als Sterilluft ausgebildet ist.

10. Verfahren zur Sterilisation von Behältern (1) mittels eines Flüssigkeits-/Gasgemisches aus einer Sterilisationsflüssigkeit und einem Gas, wonach mittels eines Ventils (5) in einer Flüssigkeits-Zuleitung (3) die Sterilisationsflüssigkeit dosiert wird, **dadurch gekennzeichnet, dass** das Gas mit der Sterilisationsflüssigkeit in einer ausgangsseitigen Ventilkammer (7b) des Ventils (5) gemischt wird.

## Claims

1. A vessel treatment machine for sterilizing containers (1) by means of a sterilization fluid, comprising at least one fluid feed line (3) and one or more gas feed lines (4; 4a, 4b), further comprising at least one mixing chamber (7b) for producing a fluid/gas mixture, and at least one valve (5) in the fluid feed line (3), **characterized in that** the mixing chamber (7b) and an outlet-side valve chamber (7b) of the valve (5) work together functionally.

2. Vessel treatment machine according to claim 1, **characterized in that** the gas feed line (4; 4a, 4b) opens out into the outlet-side valve chamber (7b).

3. Vessel treatment machine according to claim 1 or 2, **characterized in that** the outlet-side valve chamber (7b) is directly connected to a treatment head (2).

4. Vessel treatment machine according to any one of claims 1 to 3, **characterized in that** the outlet-side valve chamber (7b) is connected to the treatment head (2) with an additional second mixing chamber (9) connected in between.

5. Vessel treatment machine according to claim 4, **characterized in that** the additional second mixing chamber (9) is fed by an additional second gas feed line (4b) and a chamber feed line (10).

6. Vessel treatment machine according to any one of claims 1 to 5, **characterized in that** the gas feed line (4; 4a, 4b) comprises a Venturi nozzle (11) for accelerating the flow speed of the gas.

7. Vessel treatment machine according to any one of claims 1 to 6, **characterized in that** the valve (5) in the fluid feed line (3) is configured as a control valve.

8. Vessel treatment machine according to any one of claims 1 to 7, **characterized in that** the gas feed line (4) comprises a valve (6), in particular a control valve (6).

9. Vessel treatment machine according to any one of claims 1 to 8, **characterized in that** the sterilization fluid is realized as liquid H₂O₂ and the gas as sterile air.

10. Method for sterilizing containers (1) using a fluid/gas mixture produced from a sterilization fluid and a gas, according to which the sterilization fluid is metered by means of a valve (5) in a fluid feed line (3), **characterized in that** the gas is mixed with the sterilization fluid in an outlet-side valve chamber (7b) of the valve (5).

## Revendications

1. Machine de traitement de récipients pour la stérilisation de récipients (1) par un liquide de stérilisation, avec au moins une conduite d'amenée de liquide (3) et une ou plusieurs conduites d'amenée de gaz (4 ; 4a, 4b), comprenant de plus au moins une chambre de mélange (7b) pour la fabrication d'un mélange de liquide/gaz, et au moins une soupape (5) dans la conduite d'amenée de liquide (3), **caractérisée en ce que** la chambre de mélange (7b) et une chambre (7b) côté sortie de la soupape (5) coïncident de manière fonctionnelle.

2. Machine de traitement de récipients selon la revendication 1, **caractérisée en ce que** la conduite d'amenée de gaz (4 ; 4a, 4b) débouche dans la chambre de soupape côté sortie (7b).

3. Machine de traitement de récipients selon la revendication 1 ou 2, **caractérisée en ce que** la chambre de soupape (7b) côté sortie est directement raccordée à une tête de traitement (2).

4. Machine de traitement de récipients selon l'une quelconque des revendications 1 à 3, **caractérisée en ce que** la chambre de soupape (7b) côté sortie est raccordée à la tête de traitement (2) en intercalant une autre seconde chambre de mélange (9).

5. Machine de traitement de récipients selon la revendication 4, **caractérisée en ce que** l'autre seconde chambre de mélange (9) est alimentée par une autre seconde conduite d'amenée de gaz (4b) et une conduite d'amenée de chambre (10).

6. Machine de traitement de récipients selon l'une quelconque des revendications 1 à 5, **caractérisée en ce que** la conduite d'amenée de gaz (4 ; 4a, 4b) présente un débitmètre Venturi (11) pour l'accélération de la vitesse de fluage du gaz.

7. Machine de traitement de récipients selon l'une quelconque des revendications 1 à 6, **caractérisée en ce que** la soupape (5) dans la conduite d'amenée de liquide (3) est réalisée comme une soupape de régulation.

8. Machine de traitement de récipients selon l'une quelconque des revendications 1 à 7, **caractérisée en ce que** la conduite d'amenée de gaz (4) présente une soupape (6), en particulier une soupape de régulation (6).

9. Machine de traitement de récipients selon l'une quelconque des revendications 1 à 8, **caractérisée en ce que** le liquide de stérilisation est réalisé comme un H₂O₂ liquide et le gaz comme un air stérile.

10. Procédé de stérilisation de récipients (1) à l'aide d'un mélange de liquide/gaz composé d'un liquide de stérilisation et d'un gaz, selon lequel le liquide de stérilisation est dosé par une soupape (5) dans une conduite d'amenée de liquide (3), **caractérisé en ce que** le gaz est mélangé à du liquide de stérilisation dans une chambre (7b) côté sortie de la soupape (5).
